# EUROPÄISCHE PATENTSCHRIFT

(11) **EP 2 589 296 B1**
(45) Veröffentlichungstag und Bekanntmachung des Hinweises auf die Patenterteilung: **17.10.2018**
(21) Anmeldenummer: 12195848.2
(22) Anmeldetag: 06.05.2008
(51) Int. Cl.: A23L 2/02, A23L 2/39, A61K 31/7016, A23C 9/13, C12C 5/02, C12C 12/02, A23K 20/163, A23L 27/30, A23L 33/10, A23L 33/12, A23K 20/158

(54) **ANTIOXIDATIONSMITTEL FÜR LEBENSMITTEL**
Antioxidant for food
Agent antioxydant pour aliments

(30) Priorität: 01.06.2007 DE 102007026975
(43) Veröffentlichungstag der Anmeldung: 08.05.2013
(62) Teilanmeldung aus: 08749338.3
(73) Patentinhaber: Südzucker AG, 68165 Mannheim (DE)
(72) Erfinder: Kowalczyk, Jörg, 67304 Eisenberg/Steinborn (DE); Hausmanns, Stephan, 69126 Heidelberg (DE); Pahl, Roland, 13351 Berlin (DE)
(74) Vertreter: Wohlfahrt, Jan Günther

(56) Entgegenhaltungen:
- EP-A- 0 028 897
- EP-A- 0 809 939
- WO-A-2004/084655
- WO-A-2007/009742
- DE-A1- 2 344 252
- DATABASE CAPLUS [Online] CHEMICAL ABSTRACTS SERVICE, COLUMBUS, OHIO, US; XP002517858, gefunden im STN Database accession no. 1992:104779
- DATABASE WPI Week 199952 Thomson Scientific, London, GB; AN 1999-603566 XP002517859, & JP 2 971855 B1 (MEIJI MILK PROD CO LTD) 8. November 1999 (1999-11-08)

## Beschreibung

Die vorliegende Erfindung betrifft das technische Gebiet der Zusatz- oder Hilfsstoffe für Lebensmittel, Futtermittel, Kosmetika und Pharmazeutika besonders antioxidativ wirkende Zusatzstoffe und Antioxidantien. Die vorliegende Erfindung stellt vor allem die Verwendung von Isomaltulose zur Verminderung des Auftretens geschmacksverschlechternder Alterungskomponenten in Bier, Biermischgetränken, alkoholfreiem oder alkoholreduziertem Bier oder Biermischgetränken bereit.
Antioxidativ wirkende Zusatz- oder Hilfsstoffe für Lebensmittel, pharmazeutische oder kosmetische Mittel sind bekannt. Sie unterdrücken vor allem das Auftreten von Abbauprodukten, die bei Herstellung oder Lagerung des Lebensmittels, Futtermittels, Kosmetikums oder Pharmazeutikums beim Kontakt oxidationsempfindlicher Inhaltsstoffe mit Luftsauerstoff oder anderen oxidativ wirkenden Sub- stanzen entstehen. Im Folgenden wird auf Lebensmittel auf eine Weise Bezug genommen, dass darunter nicht nur bevorzugt Lebensmittel und Futtermittel verstanden werden, sondern auch alle weiteren im und am tierischen oder menschlichen Körper anwendbaren Mittel und Zusammensetzungen wie Kosmetika und Pharmazeutika. Als Lebensmittelzusatzstoffe wirken Antioxidantien zur Verbesserung der Lagerstabilität oder Alterungsstabilität der Lebensmittel. Sie wirken gegebenenfalls auch zusätzlich als therapeutisch und/oder prophylaktisch wirkender Wirkstoff, der seine Wirkung im tierischen oder menschlichen Körper entfaltet und dort schädliche oxidative Vorgänge unterdrückt. Ein Beispiel eines solchen Antioxidans ist Ascorbinsäure (Vitamin C). Ascorbinsäure und ihre Salze werden zum Beispiel Limonaden, Marmeladen, Kondensmilch oder Fleischprodukten beigefügt.

Antioxidationsmittel sind Lebensmittelzusatz- oder Hilfsstoffe, die in der Regel im Vergleich zu einem oxidationsempfindlichen Substrat in geringerer Konzentration vorliegen und dessen Oxidation deutlich verzögern oder verhindern. Neben der Eigenschaft, selbst ein Reduktionsmittel und damit ein Substrat oxidativer Vorgänge zu sein, können sich Antioxidationsmittel dadurch auszeichnen, dass sie Metallionen, beispielsweise zweiwertiges Eisen, welche eine katalytische Wirkung in Oxidationsreaktionen besitzen, in Form von Chelaten binden und/oder Radikal-Kettenreaktionen durch Abfangen des Starterradikals (Scavenging oder Quenchen) oder durch Abfangen eines Intermediärradikals (Chain-Breaking) unterbinden.

WO 2007/009742 A1 offenbart die Verwendung von Palatinose zur Geschmacksverbesserung von L-Carnitin in Nahrungs- oder Genussmitteln, wodurch deren Lagerstabilität erhöht werden kann.

EP 0 809 939 A1 offenbart einen Joghurt, der Palatinose und Fischöl enthält, wobei der Fischgeschmack durch Palatinose maskiert werden kann und die Lagerstabilität erhöht wird.

JP 2971855 B1 offenbart die Verwendung von Palatinose in Lebensmitteln, die den Lebensmitteln Geschmack verleiht, der sich vorteilhafterweise auch beim Erhitzen oder Lagern nicht verändert.

DE 23 44 252 A1 offenbart ein Verfahren zur Herstellung eines alkoholarmen Getränks, das mit Palatinose vergärt wird und dessen Geschmack, Farbe und Aussehen einem normalen Bier entspricht.

Viele Antioxidantien, darunter auch die Ascorbinsäure, haben auf Lebensmittel einen teilweise erheblich geschmacksveränderten Effekt. Dieser kann gezielt eingesetzt werden. So hat Ascorbinsäure einen säuerlichen, an Zitronensäure erinnernden Geschmack. In der Regel ist die antioxidative Wirkung des Antioxidationsmittels vom pH-Wert abhängig. So besitzt Ascorbinsäure hohe antioxidative Wirkung nur im Sauren. In vielen Lebensmitteln ist jedoch der säuerliche Geschmack unerwünscht oder es ist nicht möglich, ein saures Milieu einzuhalten, beispielsweise in Frischmilchprodukten. Inzwischen wurden für einige bekannte Antioxidationsmittel nachteilige gesundheitliche Auswirkungen auf den tierischen oder menschlichen Körper entdeckt. Darunter zählt auch die Ascorbinsäure sowie Schwefeldioxid und seine Salze (Sulfit, Bisulfit, Disulfit, Hydrogensulfit).

Darüber hinaus wirken viele der bekannten Antioxidationsmittel zu stark reduzierend und führen deshalb unter bestimmten Bedingungen zu unerwünschten Reaktionen mit Bestandteilen des Lebensmittels.

Viele bekannte Antioxidationsmittel, so zum Beispiel die Ascorbinsäure, sind selbst sehr oxidations- und/oder lichtempfindlich, was ihre technologische Verarbeitbarkeit erschwert.

Es besteht daher der Bedarf, weitere Substanzen der Verwendung als Antioxidationsmittel zugänglich zu machen, die bei antioxidativer Wirkung die Nachteile bekannter Antioxidationsmittel nicht aufweisen.

Ein weiterer Aspekt der Erfindung betrifft die Brauereitechnologie, vor allem die Herstellung von Bier mit hoher Lagerfähigkeit und Mitteln dazu. Bier ist als geschmacklich instabiles Lebensmittel bekannt, das einem natürlichen Alterungsvorgang unterworfen ist. Die Geschmacksstabilität stellt ein wichtiges Qualitätsmerkmal eines zur

Lagerung vorgesehenen Bieres dar. Generell wird das Ziel verfolgt, den ursprünglichen Charakter des Bieres von der Abfüllung bis zum Verbrauch beizubehalten. Der Alterungsvorgang ist vor allem durch den oxidativen Abbau von Bierinhaltsstoffen und den dabei entstehenden sogenannten "Alterungskomponenten" charakterisiert. Diese verändern den Geschmack des Bieres nachteilig. Wesentlich für den oxidativen Abbau ist der Sauerstoffeintrag nach der Gärung und bei der Abfüllung. Der molekulare Luftsauerstoff bildet reaktive Sauer- stoffformen, vor allem das Hydroxylradikal. Das Hydroxylradikal oxidiert vor allem die im Bier vorhandenen Komponenten Ethanol, freie Fettsäuren und Isohumulone zu Aldehyden und Ketonen; das Hydroxylradikal dient auch als Starterradikal für Reaktionen zu weiteren Radikalformen, woraus wiederum Aldehyde entstehen.

Bier enthält von Hause aus eine Reihe reduzierend wirkender Inhaltstoffe, die die Bildung solcher nachteiliger Oxidationsprodukte über einen gewissen Zeitraum verhindern. Diese sogenannte endogene antioxidative Aktivität oder das endogene antioxidative Potential (EAP) des Bieres verhilft dem Bier zu einer gewissen Lagerstabilität. Im Bier enthaltene antioxidativ wirkende Inhaltstoffe sind vor allem Schwefeldioxid, freie Phenole, Polyphenole oder Xanthohumole. Ist die antioxidative Kapazität dieser Inhaltstoffe erschöpft, so ist das Ende der Lagerfähigkeit des Biers erreicht. Bier hat deshalb von sich aus nur eine begrenzte Lagerfähigkeit.

Schwefeldioxid wird teilweise während der Hauptgärung durch die verwendeten Gärhefen gebildet. Die Schwefeldioxidbildung ist jedoch vom Gärverlauf und von dem verwendeten Hefestamm abhängig. Soll die endogene antioxidative Kapazität des Bieres durch Schwefeldioxid erhöht werden, muss ein spezieller Gärverlauf und eine spezielle Hefeauswahl getroffen werden; die Flexibilität bei der Ausübung der Braukunst bleibt dabei eingeschränkt.

Um die Anteile an solchen phenolischen Substanzen im Bier zu erhöhen, werden verschiedene technologische Maßnahmen bei der Bierherstellung angewendet. Solche Verfahren sind teilweise aufwändig und verändern das Brauergebnis; außerdem wird die Flexibilität bei der Ausübung der Braukunst eingeschränkt.
Ein bekanntermaßen zu Bier zusätzlich zugesetztes Antioxidationsmittel ist Schwefeldioxid. Dies wird im Allgemeinen solchen Bieren zugesetzt, die für eine längere Lagerung, beispielsweise für den Ex-ort nach Übersee, vorgesehen sind. Schwefeldioxid verändert den Geschmack des Bieres von vornherein nachteilig. Andere bekannte Oxidationsmittel wie Ascorbinsäure verändern den Geschmack des Bieres ebenfalls nachteilig.
Es ist daher Aufgabe der vorliegenden Erfindung, Mittel bereitzustellen, um die endogene antioxidative Kapazität (EAP) von Bier, Biermischgetränken und ähnlichen Brauereierzeugnissen zu erhöhen, ohne geschmackliche Nachteile und andere mit bekannten Antioxidationsmitteln verbundene Nachteile in Kauf nehmen zu müssen.
Das der Erfindung zugrundeliegende technische Problem wird gelöst durch die Verwendung von Isomaltulose zur Verminderung des Auftretens geschmacksverschlechternder Alterungskomponenten in Bier, Biermischgetränken, alkoholfreiem oder alkoholreduziertem Bier oder Biermischgetränken, wobei die Alterungskomponenten ausgewählt sind aus der Gruppe bestehend aus 2-Methylbutanol, 2-Furfural, E-2-Nonenal, Nicotinsäureethylester und gamma-Nonalacton. Bevorzugt enthält das erfindungsgemäße Antioxidationsmittel Isomaltulose als einzige antioxidativ wirkende Komponente oder es besteht daraus. In einer Variante der Erfindung ist Isomaltulose Bestandteil einer Antioxidationsmittelzusammensetzung, die neben Isomaltulose mindestens eine weitere Komponente aufweist, die die antioxidative Wirkung von Isomaltulose synergistisch unterstützt. Eine solche synergistische Wirkung steht bevorzugt im Zusammenhang mit der Komplexierung oder Chelatierung von oxidierend, katalysierend wirkenden Metallionen.

Die Beschreibung offenbart auch die Ausführungsformen (1) bis (14):
(1) Antioxidationsmittel für Lebensmittel, Futtermittel, Kosmetika oder Pharmazeutika, enthaltend Isomaltulose als die antioxidativ wirkende Komponente.
(2) Lebensmittel, Futtermittel, Kosmetikum oder Pharmazeutikum, enthaltend das in (1) charakterisierte Antioxidationsmittel.
(3) Zusammensetzung nach (2), enthaltend Isomaltulose als einzigen dem Lebensmittel hinzugegebener antioxidativ wirkender Hilfsstoff.
(4) Zusammensetzung nach (2) oder (3), enthaltend mindestens eine oxidationsempfindliche Komponente, ausgewählt aus ungesättigten Fettsäuren.
(5) Zusammensetzung nach (4), wobei die ungesättigte Fettsäure ausgewählt ist aus omega-3 Fettsäuren und omega-6 Fettsäuren.
(6) Zusammensetzung nach einer der Ausführungsformen (2) bis (5), wobei das Lebensmittel ausgewählt ist aus:
   i. Milcherzeugnissen und Milchprodukten wie Käse-, Butter-, Joghurt-, Kefir-, Quark-, Sauermilch-, Buttermilch-, Sahne-, Kondensmilch-, Trockenmilch-, Molken-, Milchzucker-, Milcheiweiß-, Milchmisch-, Milchhalbfett-, Molkenmisch- oder Milchfett-Produkte oder - Zubereitungen;
   ii. Pudding, Creme, Mousse und andere Desserts;
   iii. Milchfetterzeugnissen, Mischfetterzeugnissen, Speisefetten, Speiseölen;
   iv. Backwaren wie Brot einschließlich Kleingebäck und feinen Backwaren, Dauerbackwaren, Keksprodukten und Waffeln;
   v. Brotaufstrichen, insbesondere fetthaltigen Brotaufstrichen, Margarine-Erzeugnissen und Backfetten;
   vi. Instantprodukten und Brüherzeugnissen;
   vii. Obstprodukten oder -zubereitungen wie Konfitüren, Marmeladen, Gelees, Obstkonserven, Fruchtpulpe, Fruchtmark, Fruchtsäften, Fruchtsaftkonzentraten, Fruchtnektar und Fruchtpulver;
   viii. Cerealien, Müsli und Cerealien-Mischungen, sowie fertig zubereiteten cerealienhaltigen Produkten wie Müsli-Riegel und Frühstücksprodukten;
   ix. primär nicht-alkoholischen Getränken, Getränkegrundstoffen und Getränkepulvern, Kakaogetränken, Kakaogetränkepulvern;
   x. primär alkoholischen Getränken und Gärprodukten, Wein, Weinmischgetränken, Bier, Biermischgetränken, alkoholfreiem Bier oder Biermischgetränk, alkoholreduziertem Bier oder Biermischgetränk;
   xi. Fleischwaren und Wurstwaren;
   xii. Süßwaren wie Schokoladen, Hartkaramellen, Weichkaramellen, Kaugummi, Fondant-Erzeugnissen, Gelee-Erzeugnissen, Lakritzen, Schaumzuckerwaren, Flocken, Dragees, Komprimaten, kandierten Früchten, Krokant, Nougat-Erzeugnissen, Eiskonfekt, Marzipan, Speiseeis;
   sowie aus daraus abgeleiteter diätetischer Spezialnahrung.
(7) Zusammensetzung nach einer der Ausführungsformen (2) bis (6), die ein Futtermittel, Tiernahrung, Vormischung für Tiernahrung, stärkereiches, eiweißreiches oder fettreiches Futtermittel, Konzentratfutter oder Kraftfutter ist.
(8) Zusammensetzung nach einer der Ausführungsformen (2) bis (7), die ein omega-3 oder omega-6 Fettsäure-haltiges Milchprodukt, Joghurt oder Milchmischprodukt ist.
(9) Zusammensetzung nach einer der Ausführungsformen (2) bis (7), die ein Bier, Biermischgetränk oder alkoholfreies oder alkoholreduziertes Bier oder Biermischgetränk ist.
(10) Verwendung von Isomaltulose zur Verbesserung der Alterungsstabilität und/oder Lagerstabilität von Lebensmitteln, Futtermitteln, Kosmetika und Pharmazeutika.
(11) Verwendung von Isomaltulose zur Verbesserung der Oxidationsstabilität von Lebensmitteln, Futtermitteln, Kosmetika und Pharmazeutika.
(12) Verwendung von Isomaltulose zur Verminderung des Auftretens geschmacksverschlechternder Alterungskomponenten in Lebensmitteln, Futtermitteln, Kosmetika und Pharmazeutika.
(13) Verwendung von Isomaltulose zur Verminderung der Oxidation von ungesättigten Fettsäuren in Lebensmitteln, Futtermitteln, Kosmetika und Pharmazeutika.
(14) Verwendung nach einer der Ausführungsformen (10) bis (13), wobei das Lebensmittel ausgewählt ist aus den in (2) bis (9) charakterisierten Zusammensetzungen.

Die Erfinder fanden überraschend, dass der Zusatz von Isomaltulose in Lebensmitteln deren oxidative Stabilität deutlich verbessert. Isomaltulose wirkt als effektiver Hilfsstoff in Lebensmitteln zur Verbesserung der Lagerstabilität, Alterungsstabilität oder Oxidationsstabilität. Isomaltulose verhindert oder vermindert effektiv das Auftreten von Oxidations-Abbauprodukten, sogenannten Alterungskomponenten, die, beispielsweise in Lebensmitteln wie Bier oder bierähnlichen Getränken, durch ihre geschmacksverschlechternde und/oder gesundheitsgefährdende Wirkung die Lagerfähigkeit begrenzen. Isomaltulose wirkt protektiv für oxidationsempfindliche Nahrungsmittelkomponenten wie Farbstoffe, Geschmacksstoffe und pharmazeutische Wirkstoffe, ungesättigte Fettsäuren, darunter vor allem omega-3 und/oder omega-6 Fettsäuren und vergleichbare Fettsäuren. Ohne an die Theorie gebunden sein zu wollen, zeigt Isomaltulose überraschend eine deutlich größere und damit auch wirkungsvoll einsetzbare antioxidative Wirkung im Vergleich zu anderen bekannten reduzierenden Zuckern wie Glucose.

Die Erfindung sieht also vor, die im Lebensmittelbereich bereits bekannte Substanz Isomaltulose als Antioxidationsmittel bevorzugt für Bier, Biermischgetränke, alkoholfreies oder alkoholreduziertes Bier oder Biermischgetränke einzusetzen. Bevorzugt wird Isomaltulose als einziges dem Produkt zugegebenes Antioxidationsmittel eingesetzt.

Isomaltulose ist ein Disaccharid, welches bekanntermaßen wie Saccharose eingesetzt werden kann. Isomaltulose wird gegenwärtig hauptsächlich als Ersatz von Saccharose in bekannten Saccharosehaltigen Lebensmittelformulierungen eingesetzt. Isomaltulose wird dabei als körpergebendes Süßungsmittel ("bulk substance") verwendet. Die vorliegende Erfindung stellt hingegen eine andere Lehre bereit: Isomaltulose wird als antioxidativ wirkender Lebensmittelhilfsstoff oderzusatzstoff eingesetzt. Die Erfindung liegt somit in einem anderen technischen Anwendungsbereich, gemäß der Erfindung kann Isomaltulose auch in Zusammensetzungen und Lebensmitteln eingesetzt werden, in denen die süßende Wirkung von Isomaltulose und/oder ihre Funktion als körpergebende Komponente nicht erforderlich ist und/oder nicht zum Tragen kommt. Die erfindungsgemäße Verwendung von Isomaltulose liegt also auch außerhalb des Bereichs der Süßwaren und/oder außerhalb der kohlenhydrathaltigen Lebensmittel. Dazu zählen beispielsweise eiweiß- und/oder fettreiche Lebensmittel wie Molkereiprodukte (Käse, Joghurt etc.) oder öl- oder fetthaltige Zubereitungen (Margarine, Speiseöle etc.).

Die Beschreibung offenbart auch ein Lebensmittel, Kosmetikprodukte und pharmazeutische Präparate, welche das erfindungsgemäße Antioxidationsmittel enthält und die Verwendung von Isomaltulose als Antioxidationsmittel in einem solchen Produkt. Vorzugsweise enthält das Produkt allein Isomaltulose als einziges dem Produkt zugegebenes Antioxidationsmittel. Dabei ist nicht ausgeschlossen, dass zu der Isomaltulose mindestens eine weitere Komponente beigefügt ist, die, vorzugsweise im Zusammenhang mit einer synergistischen Wirkungsweise, den antioxidativen Effekt von Isomaltulose unterstützt oder steigert.

Ein in der Beschreibung offenbartes Lebensmittel, welches Isomaltulose als Antioxidationsmittel enthält, ist bevorzugt ausgewählt aus:
i. Milcherzeugnissen und Milchprodukten wie Käse-, Butter-, Joghurt-, Kefir-, Quark-, Sauermilch-, Buttermilch-, Sahne-, Kondensmilch-, Trockenmilch-, Molken-, Milchzucker-, Milcheiweiß-, Milchmisch-, Milchhalbfett-, Molkenmisch- oder Milchfett-Produkte oder-Zubereitungen;
ii. Pudding, Creme, Mousse und andere Desserts;
iii. Milchfetterzeugnissen, Mischfetterzeugnissen, Speisefetten, Speiseölen;
iv. Backwaren wie Brot einschließlich Kleingebäck und feinen Backwaren, Dauerbackwaren, Keksprodukten und Waffeln;
v. Brotaufstrichen, insbesondere fetthaltigen Brotaufstrichen, Margarine-Erzeugnissen und Backfetten;
vi. Instantprodukten und Brüherzeugnissen;
vii. Obstprodukten oder -zubereitungen wie Konfitüren, Marmeladen, Gelees, Obstkonserven, Fruchtpulpe, Fruchtmark, Fruchtsäften, Fruchtsaftkonzentraten, Fruchtnektar und Fruchtpulver;
viii. Cerealien, Müsli und Cerealien-Mischungen, sowie fertig zubereiteten cerealienhaltigen Produkten wie Müsli- Riegel und Frühstücksprodukten;
ix. primär nicht-alkoholischen Getränken, Getränkegrundstoffen und Getränkepulvern, Kakaogetränken, Kakaogetränkepulvern;
x. primär alkoholischen Getränken und Gärprodukten, Wein, Weinmischgetränken, Bier, Biermischgetränken, alkoholfreiem Bier oder Biermischgetränk, alkoholreduziertem Bier oder Biermischgetränk;
xi. Fleischwaren und Wurstwaren;
xii. Süßwaren wie Schokoladen, Hartkaramellen, Weickaramellen, Kaugummi, Dragees, Fondant-Erzeugnissen, Gelee-Erzeugnissen, Lakritzen, Schaumzuckerwaren, Flocken, Dragees, Komprimaten, kandierten Früchten, Krokant, Nougat-Erzeugnissen, Eiskonfekt, Marzipan, Speiseeis.

Selbstverständlich offenbart die Beschreibung auch Lebensmittel, die von den vorgenannten Lebensmitteln abgeleitet sind insbesondere diätische Spezialnahrung. Weiter offenbart die Beschreibung auch Lebensmittel, die nicht oder nicht ausschließlich für den menschlichen Verzehr gedacht oder geeignet sind; dazu zählen Futtermittel, Tiernahrung, Vormischungen für Tiernahrung, stärkereiche Futtermittel, eiweißreiche Futtermittel, fettreiche Futtermittel, Kraftfutter und Konzentratkraftfutter.

Ziel der erfindungsgemäßen Anwendung von Isomaltulose als Antioxidationsmittel sind Lebensmittel, die mindestens eine Komponente enthalten, die oxidationsempfindlich ist und Alterungsprozessen unterworfen ist, was die Haltbarkeit der Lagerfähigkeit des Lebensmittels vermindern würde. Darunter sind erfindungsgemäß Substanzen oder Substanzgemische zu verstehen, die, besonders bei der Herstellung und/oder der Lagerung der Lebensmittel, einem oxidativen Abbau unterworfen sind. Dieser oxidative Abbau wird bevorzugt durch den Kontakt mit sauerstoffhaltigen Komponenten, vor allem durch Kontakt mit Luftsauerstoff ausgelöst. Der oxidative Abbau kann außerdem durch im Lebensmittel oder in einer Lebensmittelzusammensetzung enthaltenen weiteren Substanzen, die selbst oxidativ wirken, verursacht werden. Darunter zählen beispielsweise oxidierende Säuren, Metalle in hoher Oxidationsstufe und deren Verbindungen, oxidierend wirkende Konservierungsmittel sowie andere oxidierend wirkende Sauerstoff-, Schwefel-, oder Halogen-Verbindungen. Isomaltulose als Antioxidationsmittel steigert in solchen Lebensmitteln auch die Stabilität gegenüber freien Radikalen und unterdrückt die Bildung freier Radikale.

Es ist auch ein Lebensmittel offenbart, welches Isomaltulose als, bevorzugt einziges, Antioxidationsmittel enthält und ein Milchprodukt oder Milchmischprodukt, vor allem ein Joghurt ist und als oxidationsempfindliche Komponente ungesättigte Fettsäure, vor allem omega-3 Fettsäure, omega-6 Fettsäure und/oder ähnliche enthält. Es hat sich überraschend gezeigt, dass Isomaltulose als Antioxidationsmittel den oxidativen Abbau von omega-3 Fettsäure und omega-6 Fettsäure wirksam unterdrückt. In einem Milchprodukt, besonders einem Joghurt, welches Isomaltulose enthält, wird zugesetzte omega-3 Fettsäure, omega-6 Fettsäure oder eine andere oxidationsempfindliche Komponente (siehe oben) auch bei längerer Lagerung nur in geringem Umfang abgebaut.

Der Gegenstand der Erfindung ist das Lebensmittel Bier oder davon abgewandelte Formen wie Biermischgetränk oder alkoholfreies oder alkohlreduziertes Bier oder Biermischgetränk, worin Isomaltulose als bevorzugt einziges, besonders bevorzugt einziges zugesetztes Antioxidationsmittel enthalten ist. Es hat sich überraschend gezeigt, dass ein Isomaltulose-haltiges Bier eine besonders hohe Lagerstabilität aufweist. Auch bei langer Lagerung halten sich alterungsbedingte nachteilige Geschmacksveränderungen in tolerablen Grenzen. Isomaltulose stabilisiert oxidationsempfindliche Komponenten des Biers und steigert den EAP-Wert Isomaltulose verhindert die frühzeitige Anreicherung der Alterungskomponenten, die für den sogenannten "Alterungsgeschmack" von gelagertem Bier verantwortlich sind. Zu den Alterungskomponenten im Bier, die durch das Vorhandensein von Isomaltulose effektiv unterdrückt werden können, zählen 2-Methylbutanal, 2-Furfural, E-2-Nonenal, Nicotinsäureethylester und gamma-Nonalacton. Darunter ist vor allem der oft als "Marker" für die Bieralterung bekannte Stofftrans-2-Nonenal (E-2-Nonenal) zu nennen. Nach 14-tägiger Lagerung ist sein Anteil in einem Isomaltulose-haltigen Bier gegenüber einem Kontrollbier um bis zu 20 % reduziert.

Allgemein kann die oxidative Stabilität einer Probe, beispielsweise eines Lebensmittels, mittels ESR-Spektroskopie (Elektronenspinresonanz-Spektroskopie) bestimmt werden. Diese primär für die Bestimmung der Alterungsstabilität von Bier etablierte Methode lässt sich auch auf andere oxidationsempfindliche Proben anwenden. Die ESR-Spektroskopie dient zur Bestimmung des endogenen antioxidativen Potentials (EAP-Wert) der Probe. Dies dient beispielsweise dazu, eine Aussage über die zu erwartende Lagerstabilität von Bier zu treffen. Das Verfahren beruht auf einer beschleunigten Alterung (Forciertest) der Probe bei erhöhten Temperaturen (in der Regel 60°C bis 63°C). Über die spektroskopische Detektion von Radikalfänger-Addukten wird die sogenannte "lag-time" der Probe bestimmt. Die ESR-Spektroskopie ist ein indirekter Nachweis der Radikalgenerierung in der Probe im Verlauf einer beschleunigten Alterung der Probe. Die in der Probe entstehenden Radikale sind sehr reaktiv und weisen in wässrigen Lösungen meist nur eine sehr kurze Lebensdauer auf. Es wird ein Radikalfänger, ein sogenannter "spin-trap" eingesetzt, welcher diffusible Radikale anlagern kann. Die daraus entstehenden stabilen Radikal-Addukte können anhand ihrer spektralen Charakteristika durch die ESR-Spektroskopie nachgewiesen werden. Für einen bestimmten Zeitraum, der sogenannten "lag-phase", ist die Probe aufgrund ihres endogenen antioxidativen Potentials (EAP) in der Lage, die Radikalbildung zu verhindern beziehungsweise zu verzögern. Ist das antioxidative Potential der Probe erschöpft, läuft die Radikalgenerierung ungebremst ab. Dieser Zeit- punkt wird als EAP-Wert extrapoliert. Es folgt dann ein rapider An- stieg der Signalintensität im ESR-Spektrum, welcher aus den nun gehäuft generierten "spin-trap"-Addukten resultiert.

Ein wesentliches Kriterium bei der Bestimmung der Alterungsstabilität von Lebensmitteln, vor allem Getränken ist der sogenannte BAX- Wert. Dieser ist umso günstiger, je höher der Zahlenwert ist. Der Zusatz von Isomaltulose zu Getränken, insbesondere zu Bier steigert den BAX-Wert des Getränks signifikant.

Zur Bestimmung der Alterungsstabilität von Lebensmitteln, insbesondere von Bier, wird nach der MEBAK (Mitteleuropäische Brauereianalysenkommission) das Reduktionspotential des Getränks, ins- besondere des Biers, bestimmt. Die Zugabe von Isomaltulose zum Bier steigert das reduktive Potential des Biers.

Die Beschreibung offenbart demgemäß auch die Verwendung von Isomaltulose zur Verbesserung der Alterungsstabilität Oxidationsstabilität und/oder der Lagerstabilität von Lebensmitteln, Futter- mitteln, Kosmetika und Pharmazeutika besonders von oxidationsempfindlichen Lebensmitteln, Futtermitteln,
Kosmetika und Pharmazeutika insbesondere von Bier, Biermischgetränken, Instantgetränken und Kakaoinstantgetränken; vor allem die erfindungsgemäße Verwendung von Isomaltulose zur Verminderung des Auftretens geschmacksverschlechternder Alterungskomponenten in Bier oder Biermischgetränken.

Die Beschreibung offenbart schließlich auch die Verwendung von Isomaltulose zur Verminderung der Oxidation von oxidationsempfindlichen Farbstoffen, Geschmacksstoffen, pharmazeutischen Wirkstoffen und/oder ungesättigten Fettsäuren, vor allem omega-3 Fettsäuren und ähnlichen in Lebensmitteln, insbesondere in Joghurt oder ähnlichen.

Die vorliegende Erfindung wird anhand der nachfolgenden erfindungsgemäßen und nicht erfindungsgemäßen Beispiele und Figuren näher illustriert, ohne dass diese beschränkend zu verstehen sind.

### Beispiel 1: Stabilisierung von omeaa-3 Fettsäuren in Milchprodukten (nicht erfindungsgemäß)

Es wurde die Fähigkeit von Isomaltulose untersucht, den oxidativen Abbau von omega-3 Fettsäuren, welche in einer Joghurtmatrix eingebettet sind, zu unterdrücken beziehungsweise zu vermindern.

Dazu wurde Vollfett-Joghurt (Joghurt, mild, 3,5 % Fett; Milram) eingesetzt, worin DHA CL (Lonza) als omega-3 Fettsäure eingerührt wurde. Die folgenden Joghurtzubereitungen wurden hergestellt:

| | |
|---|---|
| Ansatz 1 (erfindungsgemäß): | 5 g Isomaltulose pro 100 g; |
| Ansatz 2 (erfindungsgemäß): | 10 g Isomaltulose pro 100 g; |
| Ansatz 3: | 5 g Fructose pro 100 g; |
| Ansatz 4: | 10 g Fructose pro 100 g; |
| Ansatz 5: | 5 g Saccharose pro 100 g; |
| Ansatz 6: | 10 g Saccharose pro 100 g; |

In jeweils 240 g dieser Ansätze wurde jeweils 150 mg DHA-CL (Lonza) und etwa 40 mg einer gesättigten Fettsäure (C22:0) als interner

Standard mittels Ultraturrax eingerührt. Alle Maßnahmen erfolgten unterStickstoff-Schutzatmosphäre.

Unmittelbar nach der Herstellung der Joghurtzubereitungen wurden jeweils Nullproben gezogen und die Wiederfindungsrate der omega-3 Fettsäure in den frisch hergestellten Zubereitungen bestimmt.

Zur Bestimmung der omega-3 Fettsäuren wurden jeweils 0,85 ml der Joghurtzubereitung in ein Probenröhrchen (Eppendorf) pipetiert, gewogen und mit jeweils 1 ml tert-Butyl-methylether versetzt und intensiv geschüttelt. Nach etwa 3 min Schütteln wurden die Probenröhrchen für 3 min bei 13.000 Upm zentrifugiert. Anschließend wurden jeweils 200 µl des klaren Überstands entnommen und mit jeweils 100 µl THMS versetzt. Jeweils 1 µl dieser Lösung wurde in einen Gaschromatographen (Agilent, Typ 6890) injiziert, der in an sich bekannter Weise auf die Detektion der eingesetzten Fettsäuren optimiert war.

In allen Nullprobenansätzen wurden unterschiedslos Wiederfindungsraten von 97 bis 99 % der ursprünglich eingesetzten omega-3 Fettsäure gefunden.

Die hergestellten Joghurtzubereitungen wurden nun für 11 Tage bei 5°C gelagert. Dabei zeigte sich eine gewisse Tendenz zur Entmischung. Vor der Analyse wurden die Zubereitungen deshalb nochmals intensiv geschüttelt, wodurch die Entmischung makroskopisch rückgängig gemacht werden konnte. Die Proben wurden jeweils wie vorstehend für die Nullproben beschrieben gaschromatographisch analysiert.

Tabelle 1 zeigt die Wiederfindungsraten der omega-3 Fettsäuren in den jeweiligen Joghurtzubereitungen nach einer Lagerdauer von 11 Tagen. Überraschenderweise zeigen die Isomaltulose-haltigen Zubereitungen (Ansätze 1 und 2) die höchsten Wiederfindungsraten; es wurden Wiederfindungsraten von 95 bis 97 % der ursprünglich eingesetzten omega-3 Fettsäure gefunden. Die mit Fructose beziehungsweise Saccharose versetzten Kontrollproben zeigten deutlich geringere Wiederfindungsraten.

**Tabelle 1:**

| **Zucker** | **Anteil [g/100g]** | **Molarität [mmol/100g]** | **DHA-Ursprungswert [mg/100 g]** | **Wiederfindung DHA [%]** |
|---|---|---|---|---|
| Isomaltulose (erfindungsgemäß) | 5 | 13,8 | 26,6 | 97 |
| | 10 | 27,8 | 24,1 | 95 |
| Fructose (Vergleich) | 5 | 27,7 | 26,8 | 88 |
| | 10 | 55,5 | 24,4 | 91 |
| Saccharose (Vergleich) | 5 | 14,6 | 24,9 | 75 |
| | 10 | 29,2 | 23,2 | 78 |

Die weitere Lagerung der Joghurtzubereitungen um mehrere Wochen bestätigt die in den ersten 11 Tagen der Lagerung gefundene Wirkung der Isomaltulose, den oxidativen Abbau von omega-3 Fettsäuren zu unterdrücken.

Überraschenderweise zeigt sich, dass Fructose, die über die Keto-En(di)ol-Tautomerie als reduzierender Zucker wirkt, selbst bei höherer Molarität einen deutlich geringeren protektiven Effekt als Isomaltulose hat. Die überraschend hohe antioxidative Wirkung von Isomaltulose ist nicht allein auf die Gegenwart reaktiver Aldehydgruppen zurückzuführen. Die reduzierende Aktivität (Redox-Potential) von Aldehyd-Zuckern wurde allgemein als zu gering angesehen, als dass sich die gefundene hohe antioxidative Wirkung daraus allein herleiten könnte.

### Beispiel 2: Stabilisierung von Instantgetränken (nicht erfindungsgemäß)

Das endogene antioxidative Potential (EAP), und damit die Lagerstabilität, von Instantgetränken mit oxidationsempfindlichen Komponenten wurde untersucht. Dazu wurden Instantgetränke aus den nachfolgend beschriebenen Instantgetränkepulver-Rezepturen, die jeweils Isomaltulose beziehungsweise Saccharose als Grundlage enthielten, frisch hergestellt.

Folgende Instantpulver-Ansätze wurden hergestellt (Angaben jeweils in Gew.-%):

### Ansatz 1: "drink orange" (nicht erfindungsgemäß)

| | |
|---|---|
| Isomaltulose | 94,09 |
| Zitronensäure (wasserfrei) | 4,97 |
| Tri-Natriumcitrat (Merck) | 0,26 |
| Tri-Calciumphosphat (Merck) | 0,22 |
| Farbstoff E102 (85 %) | 0,01 |
| Farbstoff E110 (85%) | 0,016 |
| Natrium-Carboxymethylcellulose, E466 | 0,10 |
| Orangensaft-Aroma (Nr. 655228, Symrise) | 0,064 |
| Orangen-Aroma (Nr. 614756, Symrise) | 0,24 |
| Sucralose (Splenda) | 0,03 |

### Ansatz 2: "drink orange" (Vergleichsbeispiel)

| | |
|---|---|
| Saccharose | 94,12 |
| Zitronensäure (wasserfrei) | 4,97 |
| Tri-Natriumcitrat (Merck) | 0,26 |
| Tri-Calciumphosphat (Merck) | 0,22 |
| Farbstoff E102 (85 %) | 0,01 |
| Farbstoff E110 (85 %) | 0,016 |
| Natrium-Carbooximethylcelulose, E466 | 0,10 |
| Orangensaft-Aroma (Nr. 655228, Symrise) | 0,064 |
| Orangen-Aroma (Nr. 614756, Symrise) | 0,24 |

### Ansatz 3: "chocolate drink" (nicht erfindungsgemäß)

| | |
|---|---|
| Isomaltulose, gemahlen (PalatinoseTM-PA) | 79,12 |
| Kakaopulver stark entölt, dunkel GT 150 (Gerkens) 20,00 Lecithin Metarin P IP (Cargill) | 0,75 |
| NaCl (Nr. 71383, Fluka) | 0,05 |
| Vanillin (Nr. 130879, Symrise) | 0,05 |
| Sucralose (Splenda) | 0,03 |

### Ansatz 4: "chocolate drink" (Vergleichsbeispiel)

| | |
|---|---|
| Saccharose | 79,15 |
| Kakaopulver stark entölt, dunkel GT 150 (Gerkens) | 20,00 |
| Lecithin Metarin P IP (Cargill) | 0,75 |
| NaCl (Nr. 71383, Fluka) | 0,05 |
| Vanillin (Nr. 130879, Symrise) | 0,05 |

Die Ansätze wurden jeweils wie Getränkepulver benutzt und in Wasser gelöst. Dabei wurden ca. 7 bis 20 g Getränkepulver pro 200 ml fertigem Instantgetränk verwendet.

Anschließend wurden die Ansätze der beschleunigten Alterung (Forciertest) bei 63 °C unterzogen. Während der beschleunigten Alterung wurde unter Verwendung spezieller spin-trap Reagenzien in ansonsten bekannter Weise ESR-spektroskopisch das antioxidative Potential (EAP-Wert) bestimmt.

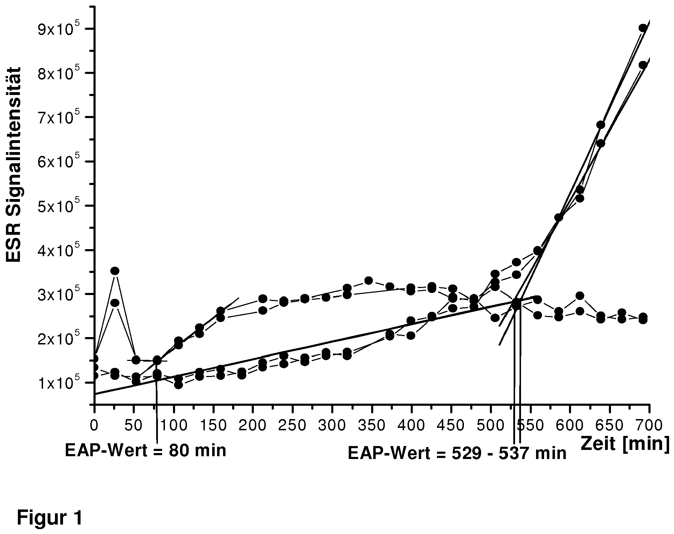

In Figur 1 sind die Messkurven der ESR-Spektroskopie für die Instantgetränke "chocolate drink" exemplarisch dargestellt.

Tabelle 2 listet die Messreihen für das Getränk "chocolate drink" aus verschiedenen Versuchsansätzen und Messläufen auf.

**Tabelle 2:**

| **EAP-Werte Instantgetränk [min]** | |
|---|---|
| **Ansatz 3** (erfindungsgemäß) | **Ansatz 4** (Vergleich) |
| 504 - 510 | <80 |
| 529 - 537 | <80 |

Isomaltulose verbesserte die oxidative Stabilität des hergestellten Instantgetränks außerordentlich.

Bei den Instantgetränken "drink orange" aus den Ansätzen 1 und 2 wurde ein generell hoher EAP-Wert gefunden (ca. 800 min oder mehr). Der erfindungsgemäße Ansatz 1 mit Isomaltulose zeigt nochmals erhöhte EAP-Werte gegenüber dem Vergleichsansatz 2.

### Beispiel 3: Bier mit verbesserter Geschmacksstabilität (erfindungsgemäß)

Zur Überprüfung der Beeinflussung der Geschmackstabilität von Bier wurde ein kommerzielles Diätbier (nahezu keine Kohlenhydrate enthaltend) mit Isomaltulose versetzt und zusammen mit der Vergleichsprobe eine definierte Bieralterung unterzogen. Es wurden folgende Analysen durchgeführt: Vergleichsverkostung, Messung des Reduktionsvermögens des Bieres, Messung der Oxidationsstabilität mittels ESR, und Gaschromatographische Bestimmung der Alterungskomponenten

In einem ersten Versuchsansatz (Ansatz 1), erfindungsgemäß wurde ein kommerzielles Diätbier mit etwa 2 g/100ml Isomaltulose versetzt. Als Vergleichsansatz diente das kommerzielle Diätbier ohne Isomaltulosezusatz (Ansatz 2).

### Ansatz 1: kommerzielles Diätbier mit Isomaltulosezusatz (erfindungsgemäß)

| | |
|---|---|
| Stammwürze [gem%] | 10,97 |
| Restextrakt scheinbar [Gew.-%] | 1,85 |
| Restextrakt wirklich [Gew.-%] | 3,55 |
| Alkoholgehalt [Vol.-%] | 4,66 |
| Bittereinheiten [BE] | 23 |

### Ansatz 2: kommerzielles Diätbier ohne Isomaltulosezusatz (Vergleichsbeispiel)

| | |
|---|---|
| Stammwürze [gem%] | 9,1 |
| Restextrakt scheinbar [Gew.-%] | 0 |
| Restextrakt wirklich [Gew.-%] | 1,66 |
| Alkoholgehalt [Vol.-%] | 4,78 |
| Bittereinheiten [BE] | 24 |

Alle Ansätze wurden 2 Wochen bei 28 °C gelagert.

### Verkostungsergebnisse

Die Verkostungsergebnisse nach zwei Wochen Lagerung bei 28 °C sind in Tabelle 3 dargestellt. (n=10 Verkoster, Idealwert: Note 5)

**Tabelle 3:**

| **Parameter** | **Ansatz 1** (erfindungsgemäß) | **Ansatz 2** (Vergleich) |
|---|---|---|
| Geruch | 4,91 | 4,89 |
| Reinheit des Geschmacks | 4,86 | 4,12 |
| Vollmundigk eit | 4,98 | 4,3 |
| Rezenz | 4,52 | 4,6 |
| Qualität der Bittere | 4,11 | 3,84 |

Das mit Isomaltulose versetzte gelagerte Bier in der Verkostung wurde als besser beurteilt. Im Geruch wird kaum ein Unterschied wahrgenommen, hingegen bestehen deutliche Unterschiede bei der Qualität des Geschmacks, der Vollmundigkeit und der Qualität der Bittere.

### Messung des Reduktionspotenzials

Nach der Methode der MEBAK wurde die Messung des Reduktionspotenzials der Biere in an sich bekannter Weise, spektralphotometrisch mittels Tannometer, durchgeführt. Es wird die Reduktion von 2,6-Dichloroindophenol über 1 min verfolgt.

**Tabelle 4:**

| **Parameter** | **Ansatz 1** (erfindungsgemäß) | **Ansatz 2** (Vergleich) |
|---|---|---|
| Reduktionsv ermögen | 51 | 45 |

Die Tabelle 4 stellt das Ergebnis dar (Mittelwerte aus Doppelbestimmung). Die Zugabe der Isomaltulose bewirkt eine Steigerung des Reduktionsvermögens um 6 Punkte. Nach MEBAK ist für helle Biere ein Wert von größer 60 (dimensionslose Zahl) als sehr gut, ein Wert von kleiner als 45 als schlecht anzusehen. Isomaltulose steigert das Reduktionspotential des eingesetzten kommerziellen Diätbiers, so dass dieses sich von der schlechten Klassifizierung deutliche abheben kann. Die Zugabe von Isomaltulose verbessert also unmittelbar die messbare Qualität von Bier.

### Bestimmung von Alterungskomponenten

Bestimmte chemische Substanzen stehen mit dem Auftreten von Alterungsgeschmack bei gelagertem Bier in Zusammenhang. Die für den Alterungsgeschmack verantwortlichen Substanzen ("Alterungskomponenten") sollen gefunden werden.

Beide Ansätze wurden für 14 Tage bei 26 °C gelagert. Anschließend wurden die Alterungskomponenten in an sich bekannter Weise chromatographisch detektiert und quantifiziert.

Tabelle 5 zeigt die chromatographisch bestimmten Alterungskomponenten nach 14 Tagen Lagerung bei 26 °C.

**Tabelle 5:**

| **Alterungskomponente [µg/L]** | **Ansatz 1** (erfindungsgemäß) | **Ansatz** 2 (Vergleich) | **Differenz [%]** |
|---|---|---|---|
| 3-Methylbutanal | 8,5 | 8,5 | 0,0 |
| 2-Methylbutanal | 2,9 | 3,4 | -17,2 |
| 2-Furfural | 250,6 | 29 | -17,4 |
| Heptanal | n.n. | n.n | 0,0 |
| Methional | n.n. | n.n | 0,0 |
| Benzaldehyd | 3,2 | 3,1 | 3,1 |
| Octanal | n.n. | n.n | 0,0 |
| Phenyl ethanal | 19,2 | 18, | 2,1 |
| E-2-Nonenal | 3,6 | 3,9 | -8,3 |
| Nicotinsäureethylester | 51,9 | 60,7 | -17,0 |
| gamma-Nonalacton | 57,4 | 62,7 | -9,2 |

3-Methylbutanal blieb unverändert, Heptanal und Methional wurden in beiden Proben nicht in nachweisbaren Mengen gefunden, daher sind auch keine Veränderungen vorhanden. Alle die meisten der analysierten Substanzen, darunter das in vielen Forschungsarbeiten als entscheidender Alterungsparameter angesehene Trans-2- Nonenal (E-2-Nonenal), sind in Gegenwart von Isomaltulose deutlich weniger gebildet worden; die Reduktion der Alterungskomponenten liegt im Bereich von 9 bis 17 %.

### Messung der oxidativen Stabilität

Eine weitere, allgemein als aussagekräftig anerkannte Analyse zur Bestimmung der Geschmacksstabilität von Bier ist die Messung der oxidativen Stabilität der Probe mittels ESR (Elektronenspinresonanzspektroskopie). Nach der Methodik von Methner und Kunz wird der BAX-Wert bestimmt. In der Bestimmung der oxidativen Stabilität des Bieres mittels ESR wird nach Methner und Kunz (Methner und Kunz, 2006 Genauere Prognosen zur oxidativen Bierstabilität mittels ESR-Spektroskopie, Brauerei Forum 2006: 7 - 9) der BAX-Wert gemessen. BAX (Beverage Antioxidant Index) wird wie folgt errechnet: BAX = Δ EAP-Wert / Δ SO₂-Gehalt [min l/mg]

Hierbei hängt der lineare Zusammenhang zwischen SO₂-Gehalt und Signalintensität der ESR Messung (EAP-Wert) von bierspezifischen Eigenfaktoren ab, die für jedes Bier unterschiedlich sind und somit in unterschiedlichen Steigungen der Kurven resultieren. Während der Messung werden die Proben mit unterschiedlichen SO₂-Gehalten dotiert und aus den entstehenden Kurven der BAX-Wert errechnet, der umso günstiger ist, je höher der Zahlenwert ist. Weiterhin wird 15 der höchste absolute Signalintensitätswert zur Beurteilung herangezogen, da er ein Maß für die während der Messung durch oxidative Vorgänge gebildeten radikalischen Substanzen darstellt.

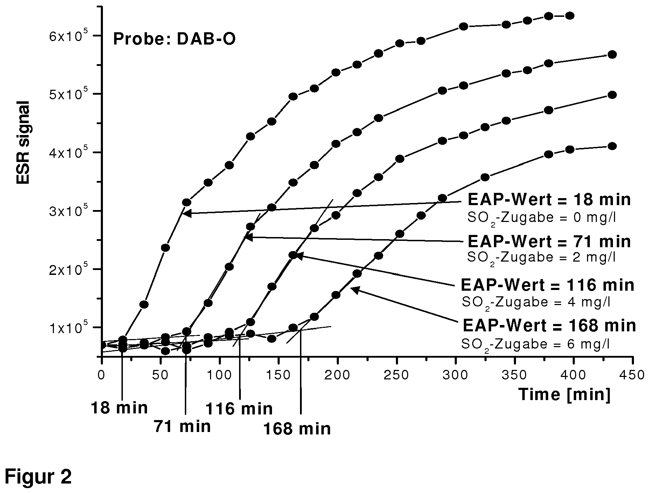

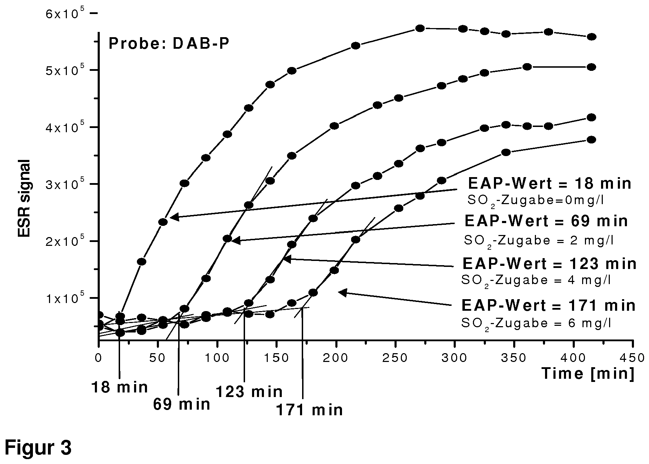

Die Figuren 2 und 3 stellen die aufgezeichneten Kurvenverläufe bei der spektroskopischen EAP-Messung dar. Aus den Kurven wurden die in Tabelle 6 dargestellten BAX-Werte errechnet.

Figur 2 zeigt die Kurvenverläufe bei der BAX-Bestimmung von Diätbier ohne Isomaltulose (Ansatz 2; Vergleichsansatz).

Figur 3 zeigt die Kurvenverläufe bei der BAX-Bestimmung von Diätbier mit Isomaltulose (Ansatz 1; erfindungsgemäß).

**Tabelle 6:**

| **Parameter** | **Ansatz 1** (erfindungsgemäß) | **Ansatz 2** (Vergleich) |
|---|---|---|
| BAX-Wert [min/mg SO₂] | 25,7 | 24,8 |

Der erfindungsgemäß erreichte BAX-Wert ist besser als bei der Vergleichsprobe ohne Isomaltulose. Die Gegenüberstellung der absoluten Höchstwerte der gemessenen Signalintensität zeigte in allen Versuchsreihen höhere Endwerte bei den Ansätzen ohne Isomaltulose. Das bedeutet, dass bei Anwesenheit der Isomaltulose insgesamt weniger radikalische Oxidationsprodukte im Bier entstehen als in der Vergleichsprobe. Figur 3 stellt die Ergebnisse dar.

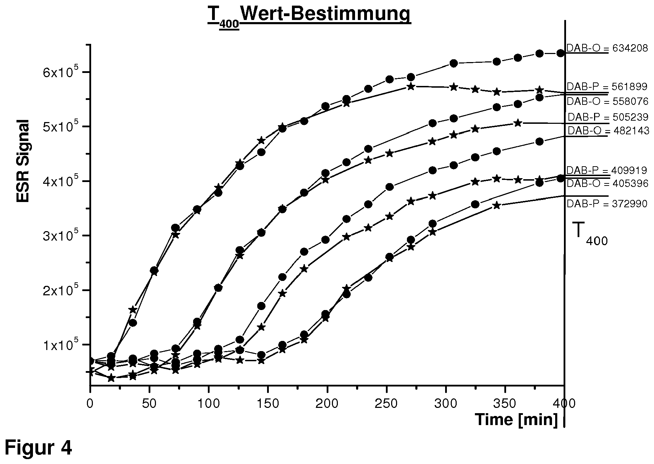

Figur 4 zeigt eine vergleichende Darstellung der Signalintensitäten der Biere mit und ohne Isomaltulose.

### Zusammenfassung der Ergebnisse

Die Zugabe von Isomaltulose wirkt sich positiv auf die Lagerungsstabilität und Geschmacksstabilität des Bieres aus. Die Zugabe von 2 g/l ist als relativ gering anzusehen, höhere Anteile verstärken die Effekte.

## Patentansprüche

1. Verwendung von Isomaltulose zur Verminderung des Auftretens geschmacksverschlechternder Alterungskomponenten in Bier, Biermischgetränken, alkoholfreiem oder alkoholreduziertem Bier oder Biermischgetränken, wobei die Alterungskomponenten ausgewählt sind aus der Gruppe bestehend aus 2-Methylbutanal, 2-Furfural, E-2-Nonenal, Nicotinsäureethylester und gamma-Nonalacton.

2. Verwendung von Isomaltulose nach Anspruch 1, wobei die Alterungskomponente E-2-Nonenal ist.

3. Verwendung von Isomaltulose nach Anspruch 2, wobei der Anteil an E-2-Nonenal nach 14-tägiger Lagerung in einem Isoma-Itulose-haltigen Bier gegenüber einem Kontrollbier um bis zu 20% reduziert ist.

## Claims

1. Use of isomaltulose for reducing the occurrence of flavour-deteriorating ageing components in beer, beer-based mixed drinks, non-alcoholic or low-alcohol beer or beer-based mixed drinks, wherein the ageing components are selected from the group consisting of 2-methylbutanal, 2-furfural, E-2-nonenal, ethyl nicotinate and gamma-nonalactone.

2. The use of isomaltulose according to Claim 1, wherein the ageing component is E-2-nonenal.

3. The use of isomaltulose according to Claim 2, wherein, after having been stored in isomaltulose-containing beer for 14 days, the percentage of E-2-nonenal is reduced by up to 20 % as compared with control beer.

## Revendications

1. Utilisation d'isomaltulose pour la réduction de l'apparition de composants de vieillissement dégradant le goût dans de la bière, des boissons de mélange de bière, de la bière ou des boissons de mélange de bière sans alcool ou à teneur réduite en alcool, dans laquelle les composants de vieillissement sont sélectionnés parmi le groupe constitué du 2-méthylbutanal, 2-furfural, E-2-nonénal, ester éthylique d'acide nicotinique et gamma-nonalactone.

2. Utilisation d'isomaltulose selon la revendication 1, dans laquelle le composant de vieillissement est le E-2-nonénal.

3. Utilisation d'isomaltulose selon la revendication 2, dans laquelle la proportion de E-2-nonénal après stockage de 14 jours dans une bière contenant de l'isomaltulose est réduite de jusqu'à 20 % par rapport à une bière témoin.
